# EUROPEAN PATENT SPECIFICATION

(11) **EP 4 561 457 B1**
(45) Date of publication and mention of the grant of the patent: **22.04.2026**
(21) Application number: 23753817.8
(22) Date of filing: 27.07.2023
(51) Int. Cl.: A61B 17/04, A61B 17/06, A61B 90/00

(54) **TISSUE CLOSURE DEVICE AND METHOD OF USING SAME**
GEWEBEVERSCHLUSSVORRICHTUNG UND VERFAHREN ZUR VERWENDUNG DAVON
DISPOSITIF DE FERMETURE DE TISSU ET SON PROCÉDÉ D'UTILISATION

(30) Priority: 28.07.2022 CN 202210898416
(43) Date of publication of application: 04.06.2025
(73) Proprietor: Cilag GmbH International, 6300 Zug (CH)
(72) Inventor: YANG, Mengli, Shanghai (CN); DING, Sheng, Shanghai (CN)
(74) Representative: van Wanrooij, Eva
(86) International application number: PCT/EP2023/070875
(87) International publication number: WO 2024/023236

(56) References cited:
- EP-A1- 3 473 189
- US-A- 6 143 004
- US-A1- 2002 005 204
- US-A1- 2008 033 459
- US-A1- 2020 060 664

## Description

### TECHNICAL FIELD

The present invention relates to the field of surgical instruments, and in particular to a tissue closure device.

### BACKGROUND OF THE INVENTION

For the existing tissue closure devices in surgical instruments, generally, the main body of the closure device is inserted into the human body or animal body tissue first, and then a suture is carried by an access needle into the tissue. After entering the tissue, the suture is fixed on a distal structure of the tissue closure device, and suturing is then completed. Because the access needle is operated outside the tissue closure device, in the process of the access needle carrying the suture into the tissue, control over the access needle may be not accurate, causing the access needle possibly to interfere with the tissue closure device, which may lead to problems such as inaccurate final arrival position of the access needle, detachment of the suture on the access needle, and failure of the suture to be accurately delivered to a predetermined position.

US 2002/0005204 A1 discloses a suture passer adapted for grasping and passing internal sutures, such as to construct a sling. The suture passer is particularly suited for use in connection with such surgery as bladder suspension procedures disclosed therein, where sutures are required to be advanced and withdrawn without direct visualization and through relatively long distances. The suture passer comprises a handle, an axially movable probe, and a probe guide having a suture channel.

US 6 143 004 A discloses a suturing device which includes a housing having an axial lumen therethrough, at least one needle having a tip and being attached to the housing and being capable of traversing along a non-linear path from a first position wherein each such needle is substantially within the housing, to a second position wherein each such needle is substantially extended from the housing, and a suture releasably attached to the housing. The suture can be captured by the tip when each such needle is in the second position and pulled from attachment to the housing when each such needle is moved to the first position. Thereafter, the suture can be released from each such needle and a knot can be formed in the suture to close a wound.

Therefore, there is a need to provide a tissue closure device and a method of using the same in order to at least partially address the aforementioned problems.

### BRIEF SUMMARY

The present invention provides a tissue closure device as recited in claim 1. Optional features are recited in the dependent claims. An access needle can be preloaded in the tissue closure device, and the tissue closure device and the access needle form an integrated unit which can be delivered to an operator conveniently. In the present invention, the needle control component can achieve accurate control over the retention and actuation processes of the access needle as such an integrated unit, such that an operator can conveniently load a suture when the access needle is held in the initial position in a predetermined orientation; and the access needle can be accurately actuated to a predetermined radial position at a predetermined depth in the process of being actuated, so as to accurately deliver the suture to a target position. The entire operating process is fast and a better operation effect can be achieved.

Also disclosed is a method of using a tissue closure device. The method is not explicitly claimed but can be carried out by a tissue closure device according to the present invention. , The method comprises:
a suture loading step comprising: loading a suture into a suture carrying portion of an access needle via a suture loading slot of a main body housing; and
a needle exiting step comprising: actuating a needle control component, such that the needle control component drives the access needle loaded with the suture to extend outwardly from a needle exit hole of the main body housing to an actuated position.

### BRIEF DESCRIPTION OF DRAWINGS

To better understand the above and other objectives, features, advantages and functions of the present invention, reference may be made to preferred embodiments shown in the accompanying drawings. The same or similar reference numerals refer to the same or similar components in the accompanying drawings. It should be understood by those skilled in the art that the drawings are intended to schematically illustrate the preferred embodiments of the invention and have no limitation on the scope of the invention as defined by the claims, and various members are not drawn to scale.
FIG. 1 is a schematic perspective view of a tissue closure device according to a preferred embodiment of the present invention, wherein an access needle is in an initial position and a stabilizer is in a folded position;
FIG. 2 is a partially enlarged schematic diagram of the structure at a suture slot of a main body housing in FIG. 1;
FIG. 3 is a schematic section view of FIG. 2, wherein the exterior structure of the main body housing is partially omitted to show the access needle;
FIG. 4 is a schematic diagram of the structure at the suture slot in FIG. 2 taken from another angle of view;
FIG. 5 is a schematic diagram of the structure at the suture slot in FIG. 2 taken from still another angle of view;
FIG. 6 is a schematic perspective view in which the main body housing in FIG. 1 is omitted;
FIG. 7 is a schematic diagram of the tissue closure device in FIG. 1 in another state, wherein the access needle is in an actuated position and the stabilizer is in an unfolded position;
FIG. 8 is a schematic perspective view in which the main body housing in FIG. 7 is omitted;
FIG. 9 is a schematic diagram showing cooperation of a needle control component and a stabilizer control component in FIG. 8;
FIG. 10 is a schematic section view of FIG. 9, which shows the interior structure of the needle control component;
FIG. 11 is a schematic exploded perspective view of the needle control component, the access needle, and a fixing pin in FIG. 10;
FIG. 12 is a schematic diagram of another embodiment of the present invention, which only shows the structure at a suture slot of a main body housing;
FIG. 13 is a schematic diagram of FIG. 12 taken from another angle of view;
FIG. 14A is a schematic diagram of still another embodiment of the present invention, which only shows the structure at and near a suture slot of a main body housing;
FIG. 14B is a schematic diagram of FIG. 14A from an angle of view taken along line A-A;
FIG. 15A and FIG. 15B are schematic diagrams of yet another embodiment of the present invention, which only show the structure at a suture slot and a suture winding portion of a main body housing, and FIG. 15A and FIG. 15B have different angles of view;
FIG. 16 is a schematic diagram of an alternative embodiment of FIG. 15, which also only shows the structure at a suture slot and a suture winding portion of a main body housing;
FIGs. 17 and 18 are schematic diagrams of a suture slot portion according to yet another embodiment of the present invention, wherein FIGs. 17 and 18 are taken from different angles of view, and FIG. 18 is a partial view; and
FIG. 19 is a section view of FIG. 18, wherein the structure of a main body housing is partially omitted to show an access needle.

### Description of reference numerals:

100 Tissue closure device
10, 610, 710, 810, 910, 1010 Main body housing
11 Housing joining line
12, 1012 Suture slot
121, 6121, 7121, 8121, 9121 Suture loading slot
1211, 10121 First slot portion
1212, 10122 Second slot portion
1212a Flared profile
122, 6122, 7122, 8122, 9122 Suture releasing slot
14 Left half housing
15 Right half housing
16, 616, 716, 816, 916, 1016 Needle exit hole
17 Needle channel
618 Notch surface
101 First window
102 Second window
20 Needle control component
21 Needle operation portion
22 Needle actuation portion
221 Radially recessed section
222 Access needle mounting hole
223 Fixing pin
2231 Fixing pin hole
224 Position-limiting slot
30 Stabilizer control component
31 Stabilizer operation portion
32 Positioning protrusion
33 Stabilizer actuation portion
40 Stabilizer
41 Shield
42 Soft rubber block
50, 1050 Access needle
51, 1051 Suture carrying portion
52 Tip of access needle
55 Notch
660, 760, 1060 Suture retaining portion
661 Protrusion portion
711 Recessed portion
761 Central portion
762 End portion
811, 911 Suture winding portion
8111 Mounting base portion
8112 Suture winding portion body
8113 Thin neck portion
813, 913 Suture accommodating portion
8131, 9131 End of suture accommodating portion
814 Recess structure

### DETAILED DESCRIPTION

A tissue closure device according to the present invention and a method of using the same are described in detail below with reference to the accompanying drawings. The embodiments described below are merely preferred embodiments according to the present invention, and on the basis of the preferred embodiments, those skilled in the art can conceive of other modes capable of implementing the present invention, which also fall within the scope of the present invention.

The present invention provides a tissue closure device for surgical procedures. First of all, it should be noted that the directional terms and position terms mentioned herein should be construed as relative directions and relative positions. For example, "axis direction" and "axial direction" and the like mentioned herein can be construed as the direction along the axis of the main body housing; "radial direction", "circumferential direction" and the like are radial and circumferential directions with respect to the axis; "distal end" and "distal" refer to the direction along the axis and away from the operator (e.g. a surgeon); and "proximal end" and "proximal" refer to the direction along the axis and close to the operator (e.g. a surgeon).

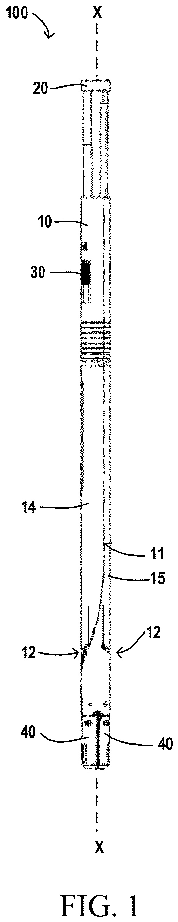

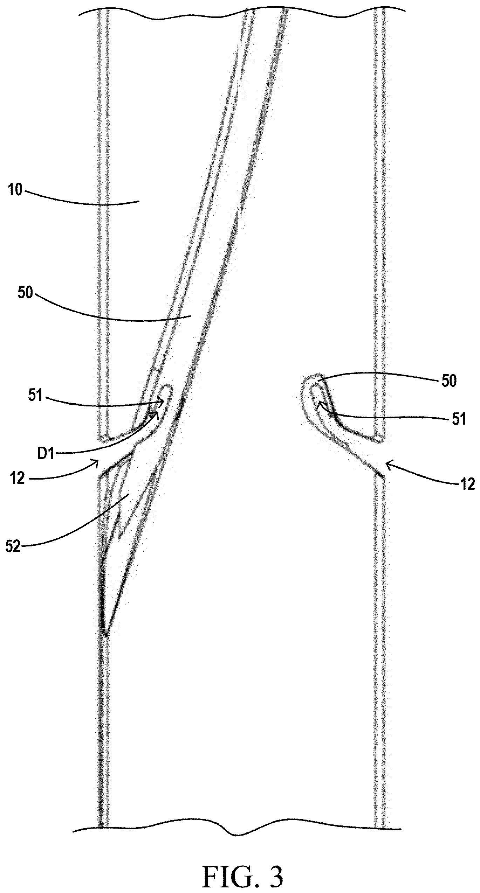

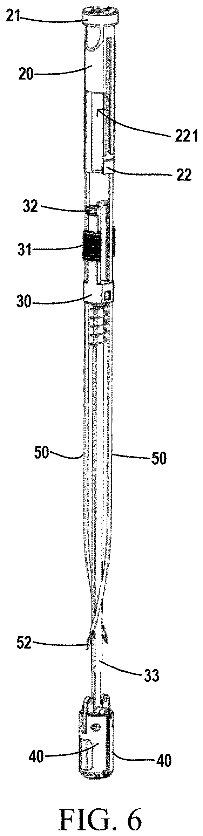

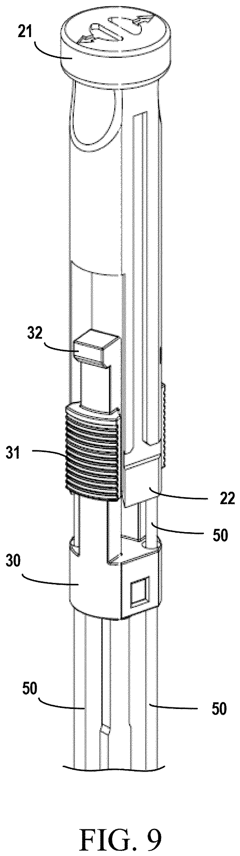

FIGs. 1 to 11 show one preferred embodiment of the present invention. First referring to FIG. 1, a tissue closure device 100 includes a main body housing 10 having a longitudinal axis, and the main body housing 10 internally defines a chamber extending along the longitudinal axis. The chamber is preloaded with an access needle 50 therein (for example, referring to the view in FIG. 6 with the main body housing 10 removed) and has a needle channel (referring to FIG. 3) for the access needle 50 to move. The access needle 50 is used to carry a suture into the tissue of a patient. The tissue closure device 100 further includes a needle control component 20 mounted at a proximal portion thereof, and the needle control component 20 is fixedly connected to a proximal end of the access needle 50. The needle control component 20 can move axially relative to the main body housing 10, thereby controlling the access needle 50 to move from an initial position to an actuated position.

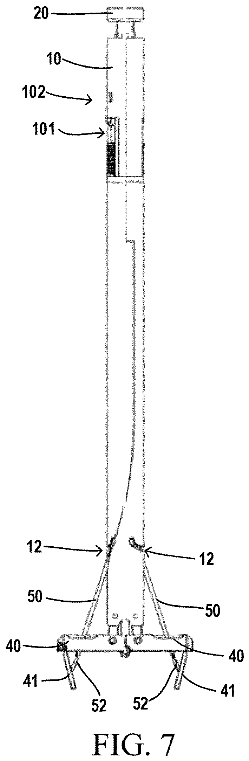

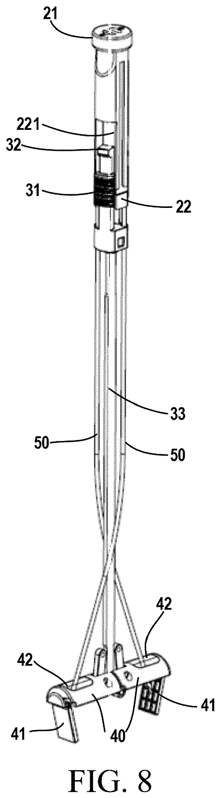

Reference may be made to FIGs. 1 to 6 for the state of the access needle 50 in the initial position, and reference may be made to FIGs. 7 and 8 for the state of the access needle 50 in the actuated position. When the access needle 50 is in the initial position relative to the main body housing 10, the access needle 50 is entirely located in the chamber of the main body housing 10, and a distal end 52 of the access needle 50 is in the most proximal position relative to the main body housing 10. When the access needle 50 is in the actuated position relative to the main body housing 10, the distal end 52 of the access needle 50 is located outside the main body housing 10 and the distal end 52 of the access needle 50 is in the most distal position relative to the main body housing 10.

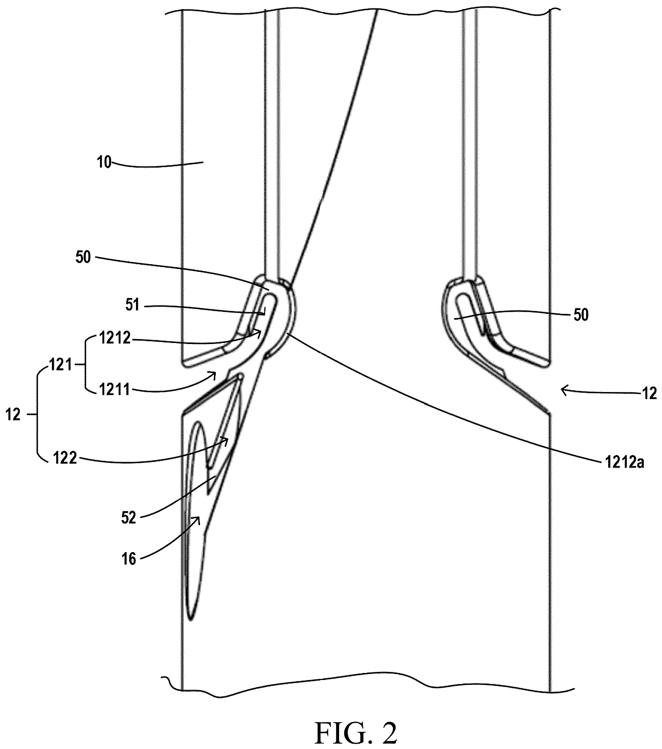

Also referring to FIGs. 2 to 5, a suture slot 12 that communicates the chamber of the main body housing 10 with the outside is provided on a sidewall of the main body housing 10, and the suture slot 12 at least partially extends along the radial direction of the main body housing 10. The access needle 50 is formed with a suture carrying portion 51 close to the distal end 52 thereof, and the suture carrying portion 51 is also in the form of a slot structure extending at least partially proximally along the extending direction of the access needle 50. The needle control component 20 can hold the access needle 50 in the initial position in a predetermined orientation. When the access needle 50 is held in the initial position, the distal end 52 of the access needle 50 is located in the chamber. The "predetermined orientation" means that the orientation of the access needle in the initial position enables the suture slot 12 of the main body housing 10 and the suture carrying portion 51 of the access needle 50 to be at least partially communicated, such that the operator can load a suture into the suture carrying portion 51 of the access needle 50 via the suture slot 12 of the main body housing 10 (it can be appreciated that if the access needle 50 is rotated with respect to the predetermined orientation, the suture carrying portion 51 may not communicate with the slot 12, resulting in failure to successfully load the suture). For example, the inner wall of the suture slot 12 is defined to have a flat surface, such that the access needle 50 is fit to the flat surface and prevented from rotating in the suture slot 12, thus ensuring partial communication between the suture carrying portion 51 and the suture slot 12.

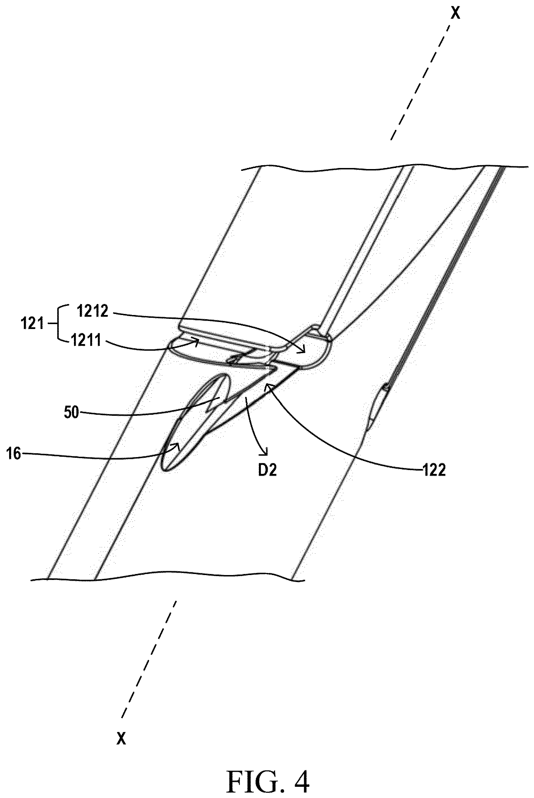

The suture slot 12 may include, for example, a suture loading slot 121 and a suture releasing slot 122. The suture releasing slot 122 may, for example, communicate at a distal end of the suture loading slot 121 and a distal end of the suture releasing slot 122 may further communicate with a needle exit hole 16. The needle control component 20 can control the access needle 50 to extend to an actuated position at a predetermined depth. In the process of the access needle 50 moving from the initial position to the actuated position, the distal end 52 of the access needle 50 will extend through the needle exit hole 16 and exit the main body housing 10. The portion serving as the suture loading slot 121 of the suture slot 12 is used to load a suture into the suture carrying portion 51 from the outside of the tissue closure device 100 when the access needle 50 is held in the initial position in the predetermined orientation, wherein the movement direction of the suture in this process is shown by, for example, an arrow D1 in FIG. 3. The portion serving as the suture releasing slot 122 of the suture slot 12 is used to allow the suture to exit the tissue closure device via the suture releasing slot 122 after the access needle has been moved to the actuated position via the needle exit hole 16, wherein the movement direction of the suture in this process is shown by, for example, an arrow D2 in FIG. 4.

Further, the suture loading slot 121 has a first slot portion 1211 and a second slot portion 1212. The first slot portion 1211 extends at least partially towards the axis from the outside to the second slot portion 1212, and the second slot portion 1212 extends at least partially axially proximally from the first slot portion 1211, wherein the second slot portion 1212 and the suture carrying portion 51 are at least partially aligned. For example, as shown in FIGs. 2 and 3, when the access needle 50 is in the initial position, the extending directions of the second slot portion 1212 and the suture carrying portion 51 of the access needle 50 are substantially the same, as can be seen from a side view of the tissue closure device 100. More specifically, in this embodiment, the first slot portion 1211 extends both radially inward and axially proximally, and the second slot portion 1212 also extends radially inward and axially proximally. An included angle between the extending direction of the second slot portion 1212 and the longitudinal axis is less than an included angle between the first slot portion 1211 and the longitudinal axis.

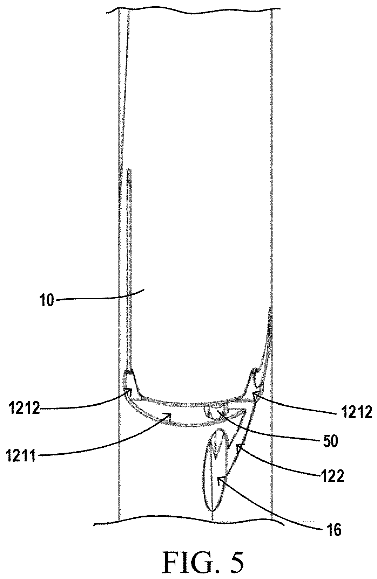

Also referring to FIGs. 4 and 5, the first slot portion 1211 constitutes an elongated opening formed on the sidewall in a direction perpendicular to the longitudinal axis, and two ends of the elongated opening respectively extend proximally and towards the axis to jointly define the second slot portion 1212. The extending directions of the suture releasing slot 122 and the second slot portion 1212 are substantially the same, and the suture releasing slot 122 smoothly transitions from the second slot portion 1212 to the needle exit hole 16. It can be clearly seen from FIGs. 4 and 5 that, the needle exit hole 16 is an opening fitting the shape of the access needle 50, and the opening may have, for example, a round or oval contour.

Because the suture slot 12 is formed on the main body housing 10 made of a rigid material in this embodiment, the suture slot 12 needs to have a certain width to allow the suture to pass through. For example, the width of the suture loading slot 121 is greater than or equal to the width of the suture carrying portion 51 of the access needle 50. Preferably, the suture loading slot 121 may have a clearance design at the boundary, so that after the suture enters the suture loading slot 121, the suture carrying portion 51 can also be pressed to drive the distal end of the access needle 50 to move in the suture slot 12 in a very small range, thus more accurately completing suture loading of the access needle 50. The clearance design may include, for example, a flared profile 1212a relative to the center of the suture loading slot, that is formed on the boundary of the second slot portion 1212 of the suture loading slot 121, and the flared profile 1212a may be, for example, a curved segment. The clearance design may also have other forms.

Referring to FIGs. 1, and 6 to 8 as a whole, in this embodiment, two access needles 50 are provided, and the proximal ends of the two access needles 50 are fixed on the same needle control component 20, so that the needle control component 20 controls the two access needles 50 simultaneously. The access needle 50 is easily bendable and flexible, and is made of, for example, a plastic material. The chamber in the main body housing 20 includes a needle channel which is configured to enable the access needle 50 to move therein in a predetermined pose. In other words, the form of the needle channel determines the form of the access needle 50. It can be seen from FIGs. 6 and 8 that, in the initial position, each access needle 50 may include, for example, a straight segment and a curved segment located at the distal end of the straight segment, and the curved segment extends distally partially about the longitudinal axis. Then, it can be understood that, in fact, it is the needle channel that has such straight and curved segments. In the actuated position, the access needle 50 may include two straight segments and a curved segment between the two straight segments. The curved segment extends partially about the longitudinal axis, the straight segment located proximally of the curved segment extends axially, and the straight segment located distally of the curved segment extends both axially and radially. Likewise, it can be understood that, in fact, it is the needle channel that has such straight and curved segments (of course, expect for the portions outside the main body housing).

Also referring to FIG. 8, under the operation of the needle control component 20 and the position-limiting effect of the needle channel, when the distal ends 52 of the access needles 50 reach the actuated position, a connecting line between the distal ends 52 of the two access needles 50 is in a radial direction with respect to the axis, which is, for example, also the extending direction of a stabilizer 40 (which will be described in detail later).

The two needle channels are symmetrical about the axis at any axial position. As shown in FIGS. 6 and 8, such arrangement enables the portions of the two access needles 50 at any axial position to be symmetrical about the longitudinal axis. Correspondingly, two perforated structures are correspondingly provided on the sidewall of the main body housing 10, and each of the perforated structures includes a suture slot 12 and a needle exit hole 16. The two perforated structures are substantially symmetrical about the axis. For example, the corresponding parts of the two perforated structures are symmetrical about the axis. Further, for example, the respective suture loading slots 121 of the two perforated structures are symmetrical about the axis, the respective suture releasing slots 122 are symmetrical about the axis, and the respective needle exit holes 16 are symmetrical about the axis.

Referring to FIGs. 1, 7 and 8, the tissue closure device 100 further includes a stabilizer 40, and a stabilizer control component 30 mounted on the main body housing 10 close to a proximal end thereof. The stabilizer 40 includes, for example, at least two wing portions that are located on a distal end of the main body housing 10. The stabilizer control component 30 can move axially with respect to the main body housing 10 in response to operation of an operator, thereby driving the at least two wing portions to be actuated between a folded position and an unfolded position. For example, when the stabilizer control component 30 moves distally relative to the main body housing 10, the stabilizer 40 is actuated from the folded position to the unfolded position. In the state shown in FIG. 1, the stabilizer 40 is in the folded position. The stabilizer 40 in the folded position extends substantially axially distally from the main body housing 10, and the radial dimension of the stabilizer 40 in the folded position is approximately equal to the outer diameter of the main body housing 10. In the states shown in FIGs. 7 and 8, the stabilizer 40 is in the unfolded position, and the extending direction of the stabilizer 40 in the unfolded position is substantially perpendicular to the longitudinal axis. The stabilizer 40 may be used as, for example, a depth limiting mechanism. When the distal end of the tissue closure device 100 extends to approximately a predetermined depth within the patient, the stabilizer control component 30 may be operated to unfold the stabilizer 40. The proximal surface of the unfolded stabilizer 40 is fit to the inner wall of the tissue of the patient, thereby controlling the extension of the tissue closure device 100 to the current depth within the patent.

A soft rubber block 42 may be provided on the stabilizer 40. In the process of the access needle 50 moving from the initial position to the actuated position, the distal end 52 of the access needle 50 will pass through the soft rubber block 42 and reach the distal position of the stabilizer 40. Preferably, in order to prevent the distal end 52 of the access needle 50 from piercing the tissue of the patient, a shield 41 extending distally from the stabilizer 40 may be provided at a radial outer side of the stabilizer 40. When the access needle 50 is in the actuated position, the shield 41 provides protection at a radial outer side of the distal end 52 of the access needle 50. Additionally or alternatively to the above preferred arrangements, this embodiment may also have preferred arrangements in some aspects. The preferred arrangements will be discussed in detail below with reference to FIGs. 1, and 7 to 11.

For example, this embodiment may have some preferred arrangements in terms of the form of the needle control component 20, the joining manner between the needle control component 20 and the main body housing 10, and the connection manner between the needle control component 20 and the access needle 50. The needle control component 20 has a needle operation portion 21 located outside the main body housing 10 for a user to operate, and a needle actuation portion 22 located in the chamber and connected to the access needle 50. The chamber of the main body housing 10 may have a proximal opening from which the needle operation portion 21 extends to form a button structure for the operator to press. Further, a position-limiting structure is provided at the connection position between the needle control component 20 and the main body housing 10, and the position-limiting structure only allows the needle control component 20 to move axially relative to the main body housing 10 and prevents the needle control component 20 from rotating or translating in other directions relative to the main body housing 10.

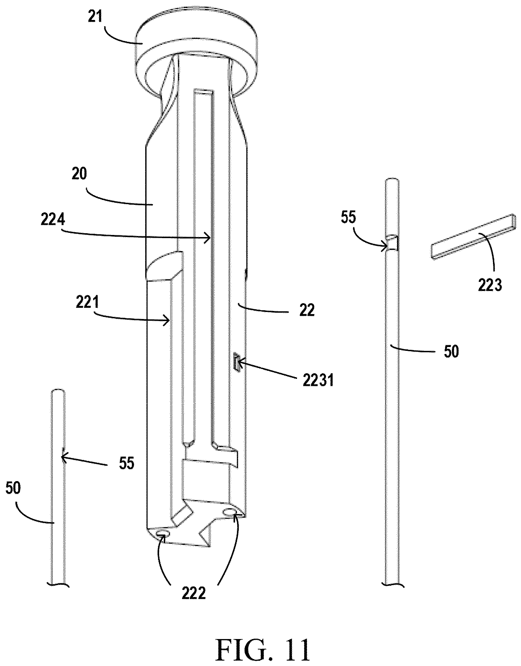

The position-limiting structure includes, for example, an axially extending position-limiting slot 224 (as shown in FIG. 11) located on an outer surface of the actuation portion, and a protrusion located on an inner surface of the main body housing 10 and having a width matching that of the position-limiting slot 224. In this embodiment, the main body housing 10 includes a left half housing 14 and a right half housing 15, and the left half housing 14 and the right half housing 15 are butted together along a direction transverse to the longitudinal axis, with a housing joining line 11 formed at the connection position therebetween. The left half housing 14 and the right half housing 15 are respectively provided with protruding halves at portions thereof adjacent to each other. The protruding halves of the left half housing 14 and the right half housing 15 are butted together to jointly define the protrusion.

It can be understood that, in this embodiment, the protrusion can slide along the position-limiting slot 224 so that the needle control component 20 moves from a first position (for example, a proximal position) to a second position (for example, a distal position) relative to the main body housing 10. When the needle control component 20 is located at the first position, the needle control component 20 holds the access needle 50 in the initial position in a predetermined orientation. When the needle control component 20 moves from the first position to the second position, the access needle 50 is driven to move from the initial position to the actuated position at a predetermined depth.

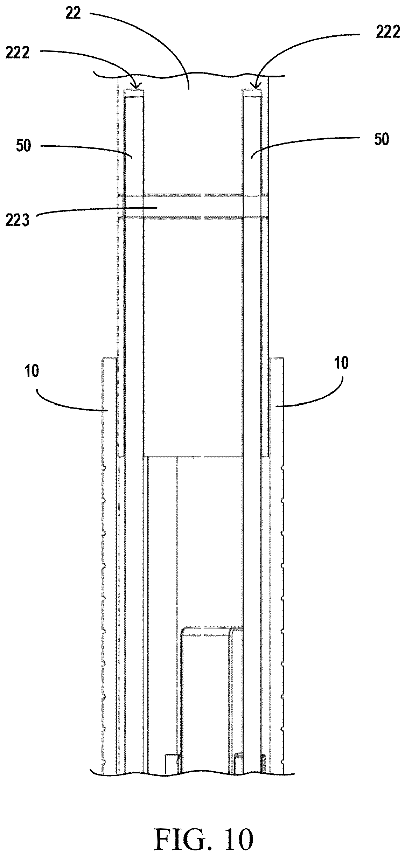

Referring to FIGs. 10 and 11, the needle actuation portion 22 has two holes 222 that are open distally and extend axially, and the inner diameter of each hole 222 matches the outer diameter of one access needle 50. And a fixing pin 223 transverse to the longitudinal axis is provided in the needle control component 20 and the fixing pin 223 is connected between the two holes 222. Each access needle 50 may be provided with a notch 55 for receiving the fixing pin 223, and the fixing pin 223 is engaged with and fixed to the notch 55. That is to say, one fixing pin 223 is joined to two access needles 50 simultaneously, so that the two access needles 50 are fixed to the needle actuation portion 22 simultaneously. The needle actuation portion 22 is provided with a fixing pin hole 2231 for insertion of the fixing pin 223 in an assembly stage.

The wall surface of the notch 55 may include a flat surface, so as to enhance the joining stability between the fixing pin 223 and the access needle 50. The notch 55 may extend in the middle portion of the access needle 50, and may also extend all the way to the proximal end of the access needle 50. It should be noted that, the notch is only an example of the "mating portion", and the mating portion may also have other forms in other embodiments that are not shown. In some embodiments, the fixing pin may not be provided, and the mating portion is directly molded together with the needle control component.

This embodiment may also have some preferred arrangements in terms of the connection manner between the stabilizer control component 30 and the main body housing 10, and the connection manner between the stabilizer control component 30 and the needle control component 20. For example, the sidewall of the main body housing 10 is provided with a first window 101 and a second window 102 located proximally of the first window 101 (referring to FIG. 7). The stabilizer control component 30 includes a stabilizer operation portion 31 exposed through the windows, a positioning protrusion 32 located proximally of the stabilizer operation portion 31 and fixed relative to the stabilizer operation portion 31, and a stabilizer actuation portion 33 located distally of the stabilizer operation portion 31. A radial outer portion of the stabilizer operation portion 31 has an axial dimension smaller than that of the first window 101 so as to be able to axially slide in the first window 101. The size of a radial outer portion of the positioning protrusion 32 matches that of the second window 102. When the positioning protrusion 32 is placed in the second window 102, the stabilizer control component 30 is locked relative to the main body housing 10, for example, as shown in FIG. 1. When the positioning protrusion 32 is located in the second window 102, the operator is allowed to press the positioning protrusion 32 radially inwardly, and the positioning protrusion 32 is biased inwardly to disengage from the second window 102 to unlock the stabilizer control component. Subsequently, the positioning protrusion 32 can be actuated distally together with the stabilizer operation portion 31. When the stabilizer control component 30 actuates the stabilizer 40 to the unfolded position, the positioning protrusion 32 is positioned in the first window 101 together with the stabilizer operation portion 31, as shown in FIGs. 7 and 8. Preferably, the axial dimension between the most proximal end of the positioning protrusion 32 and the most distal end of the stabilizer operation portion 31 is just equal to the axial dimension of the first widow 101, such that the stabilizer control component 30 cannot move axially relative to the main body housing 10 when the positioning protrusion 32 is located in the first window 101 together with the stabilizer operation portion 31.

Further, there are two stabilizer operation portions 31. The stabilizer control component 30 further includes a stabilizer actuation portion 33 fixedly connected at the distal ends of the two stabilizer operation portions 31. A radially recessed section 221 (as shown in FIG. 11) is provided on a sidewall of the needle control component 20, and at least a radial inner portion of the stabilizer operation portion 31 is correspondingly mounted at the radially recessed section 221. The needle control component 20 is partially located between the two stabilizer operation portions 31 and is completely located proximally of the stabilizer actuation portion 33.

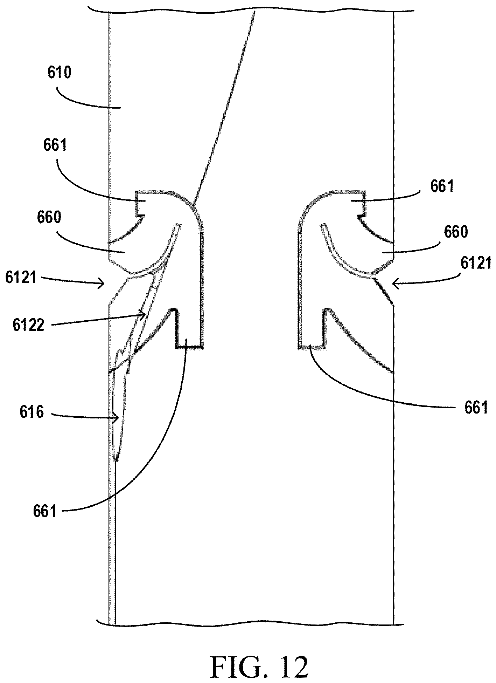

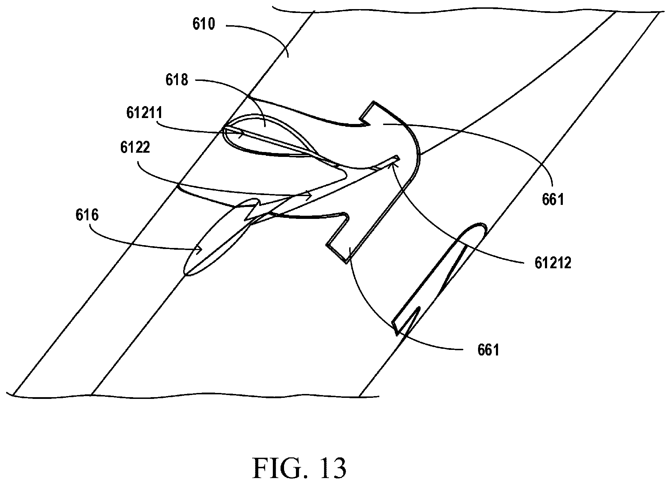

FIGs. 12 and 13 show the structure at the suture slot of the tissue closure device according to another preferred embodiment of the present invention. A main body housing 610 includes a rigid sidewall and a suture retaining portion 660 that can provide greater friction. The suture retaining portion 660 is made of, for example, a flexible, plastic, or elastic material. In an embodiment, the suture retaining portion 660 may be made of a soft rubber. The suture retaining portion 660 can hold the suture carried by the access needle in place by means of friction when the access needle is in the initial position. For example, a window is formed on the rigid sidewall, and the suture retaining portion 660 is mounted at the window and constitutes a part of the sidewall of the main body housing 610, wherein at least a part of the suture slot is formed on the suture retaining portion 660.

As shown in FIGs. 12 and 13, a suture loading slot 6121 and a suture releasing slot 6122 are both formed on the suture retaining portion 660, and a needle exit hole 6122 is jointly defined by the suture retaining portion 660 and the rigid sidewall. In this embodiment, the suture loading slot 6121 also includes a first slot portion 61211 and a second slot portion 61212, and notch surfaces 618 that function as a guide structure are formed at an inlet of the first slot portion 61211. The notch surfaces 618 are provided proximally and distally of the inlet respectively, to facilitate loading of the suture from the outside via the suture slot into the suture carrying portion of the access needle.

In this embodiment, because the suture retaining portion 660 can provide friction to the suture, a suture slot 612 formed on the suture retaining portion 660 can have a smaller width. For example, the width of the suture slot 612 can be smaller than the width of the suture slot in the embodiment shown in FIGs. 1 to 11. Further, the width of the suture slot 612 can be even smaller than or equal to the width of the suture, such that when the suture enters the suture slot 612, an interference fit is created between the suture and the suture slot 612, and a slight deformation may occur at the suture slot 612. This slight deformation does not affect the loading of the suture, but can help to clamp the suture, avoiding the suture from coming off. It can be understood that, if the suture slot is partially defined by the suture retaining portion (for example, the suture is jointly defined by the suture retaining portion and the rigid sidewall), the suture slot can still have such a smaller width.

Preferably, the suture retaining portion 660 is provided with a protrusion portion 661 protruding along the sidewall of the main body housing 610, a corresponding recess portion is provided at the window of the rigid sidewall, and the protrusion portion 661 and the recess portion cooperate to secure the suture retaining portion 660 and the rigid sidewall. Additionally or alternatively to this solution, a protrusion portion protruding along the sidewall of the main body housing is provided at the window of the rigid sidewall, a corresponding recess portion is provided at the edge of the suture retaining portion, and the protrusion portion and the recess portion cooperate to secure the suture retaining portion and the rigid sidewall.

It should be noted that, in addition to the foregoing structures, this embodiment is identical to or similar to the embodiment shown in FIGs. 1 to 11, and the description of the embodiment in FIGs. 1 to 11 should also be regarded as description of this embodiment.

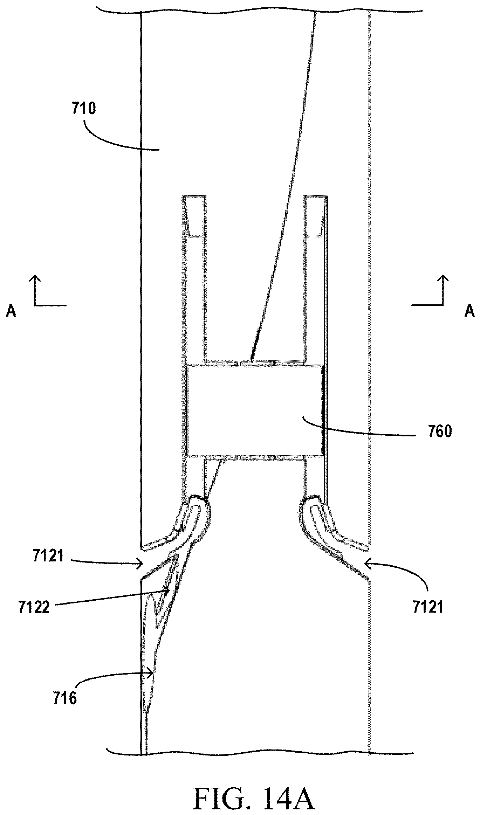

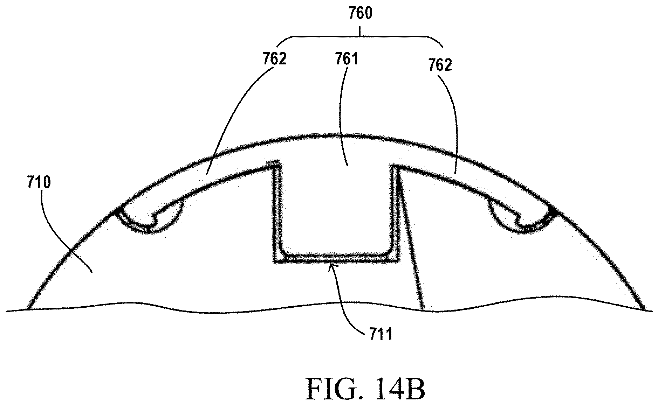

FIGs. 14A and 14B show still another preferred embodiment of the present invention. In this embodiment, a suture retaining portion 760 is different from the suture retaining portion in FIGs. 12 and 13. In the embodiment shown in FIG. 14A, a suture loading slot 7121, a suture releasing slot 7122, and a needle exit hole 716 are provided on the sidewall of the main body housing 710, and the structural forms of the suture loading slot 7121, the suture releasing slot 7122, and the needle exit hole 716 are identical to or similar to those in FIGs. 1 to 11. In FIG. 14, the suture retaining portion 760 is mounted on the rigid sidewall and independent of the suture loading slot 7121, the suture releasing slot 7122, and the needle exit hole 716. The suture retaining portion 760 allows the suture to enter between the suture retaining portion 760 and the rigid sidewall so as to clamp the suture between the suture retaining portion 760 and the rigid sidewall. Preferably, the suture retaining portion 760 may be mounted proximally of the suture loading slot 7121, and located between the two suture loading slots 7121 circumferentially.

Preferably, the outer surface of the suture retaining portion 760 is flush with the outer surface of the rigid sidewall. It can be understood that, in order to achieve this feature, a recessed portion 711 will be provided on the rigid sidewall, and the suture retaining portion 760 has a matching shape with the recessed portion and is inserted therein. More preferably, referring to FIG. 14B, the suture retaining portion includes a central portion 761 and end portions 762 located at both circumferential ends of the central portion 761, and the inner surface of the central portion 761 protrudes further inwardly toward the longitudinal axis relative to the inner surfaces of the end portions 762.

It should be noted that, in addition to the foregoing structures, this embodiment is identical to or similar to the embodiment shown in FIGs. 1 to 11, and the description of the embodiment in FIGs. 1 to 11 should also be regarded as description of this embodiment.

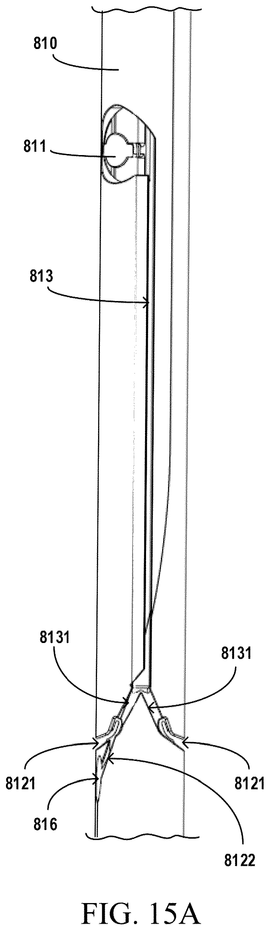

FIGs. 15A to 16 show some other preferred embodiments of the present invention, and mainly illustrate two forms of a suture winding portion provided on a main body sidewall. It should be noted that, the suture winding portion in FIGs. 15A to 16 may be applied to the embodiments in FIGs. 1 to 14.

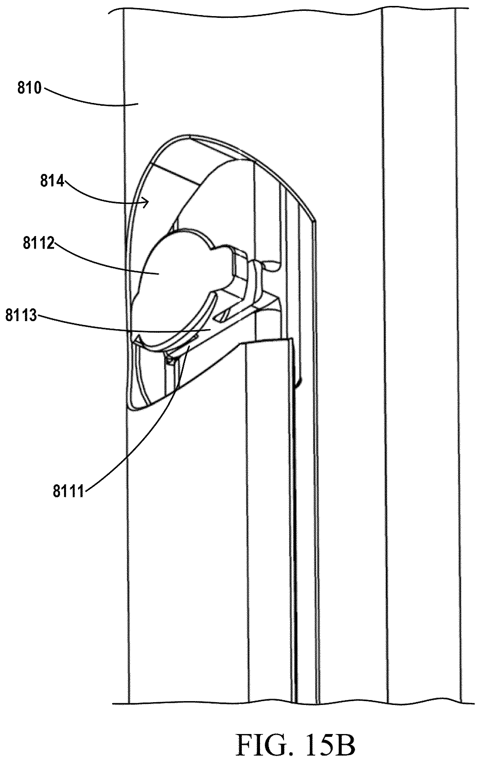

Referring to FIG. 15A, a suture loading slot 8121, a suture releasing slot 8122, and a needle exit hole 816 that are similar to those in the embodiment of FIGs. 1 to 11 are provided on the sidewall of a main body housing 810. The main body housing is further provided with a recessed and externally open suture accommodating portion 813 that extends parallel to the longitudinal axis, the distal end of the suture accommodating portion 813 communicates with the suture loading slot 8121, and the proximal end of the suture accommodating portion 813 is provided with a suture winding portion 811 for loading the suture. Two branches 8131 are formed at the distal end of the suture accommodating portion 813, so as to communicate with the two suture loading slots 8121, respectively. It can be seen from FIG. 15B that, the suture winding portion 811 is mounted in a recess structure 814 on the main body housing 810, and the suture winding portion 811 is formed into a round button and includes a mounting base portion 8111 directly joined to the main body housing 810 and a suture winding portion body 8112 located on the outer side of the mounting base portion 8111. The mounting base portion 8111 and the suture winding portion body 8112 are connected by a thin neck portion 8113, and the suture is wound around the thin neck portion 8113.

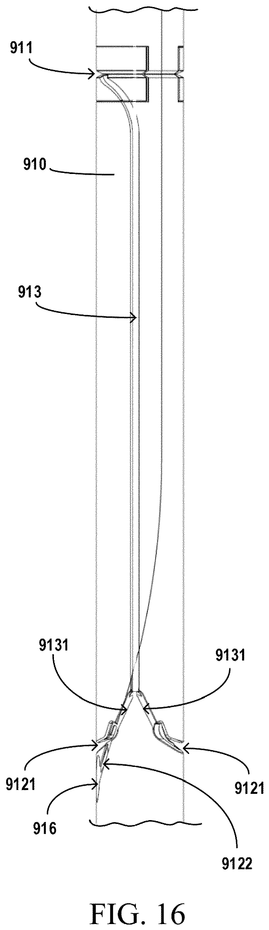

Referring to FIG. 16, a suture loading slot 9121, a suture releasing slot 9122, and a needle exit hole 916 that are similar to those in the embodiment of FIGs. 1 to 11 are provided on a main body housing 910. The main body housing 910 is further provided with a recessed and externally open suture accommodating portion 913 that extends parallel to the longitudinal axis, the distal end of the suture accommodating portion 913 communicates with the suture loading slot 9121, and the proximal end of the suture accommodating portion 913 is provided with a suture winding portion 911 for loading the suture. Two branches 9131 are formed at the distal end of the suture accommodating portion 913, so as to communicate with the two suture loading slots 9121, respectively. The suture winding portion 911 may be a recessed groove extending along the entire circumference.

It should be noted that, various arrangements in this embodiment may be identical to or similar to the embodiment shown in FIGs. 1 to 11, and the description of the embodiment in FIGs. 1 to 11 should also be regarded as description of this embodiment.

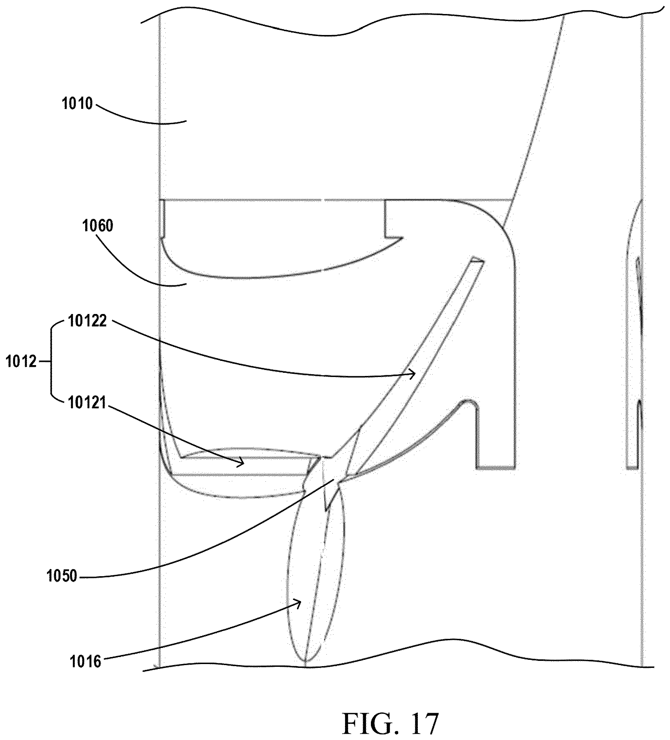

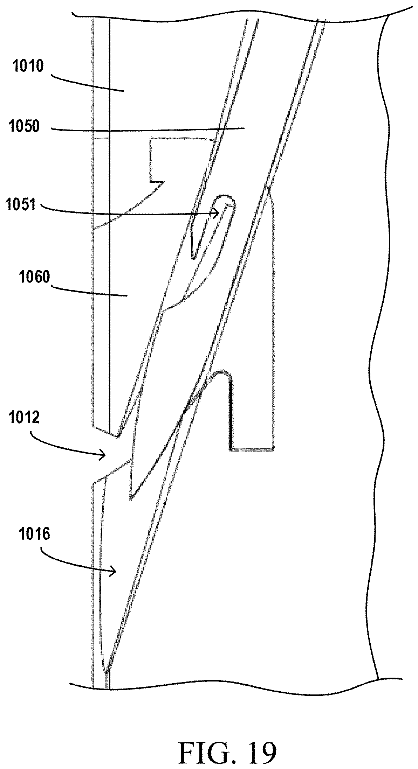

FIGs. 17 to 19 show still another embodiment of the present invention. In this embodiment, the form of the suture slot on the main body sidewall is different from that shown in FIGs. 1 to 16. Referring to FIGs. 17 to 19, a suture slot 1012 is an integrated slot serving as both a suture loading slot and a suture releasing slot. The suture slot 1012 is configured to allow a suture to be loaded from the outside of the tissue closure device via the suture slot 1012 into a suture carrying portion 1051 of an access needle 1050 and to allow the suture to disengage from the tissue closure device via the suture slot 1012. The suture slot 1012 directly communicates with a needle exit hole 1016. Further, the suture slot 1012 is formed on a suture retaining portion 1060 on the main body sidewall, and the needle exit hole 1016 is jointly defined by the suture retaining portion 1060 and the rigid sidewall. The suture slot 1012 also includes a first slot portion 10121 serving as a suture loading inlet, and a second slot portion 10122 extending proximally from the first slot portion 10121 and towards the axis. When a suture is loaded, the suture needs to move proximally along the second slot portion 10122, till the suture reaches the suture carrying portion 1051.

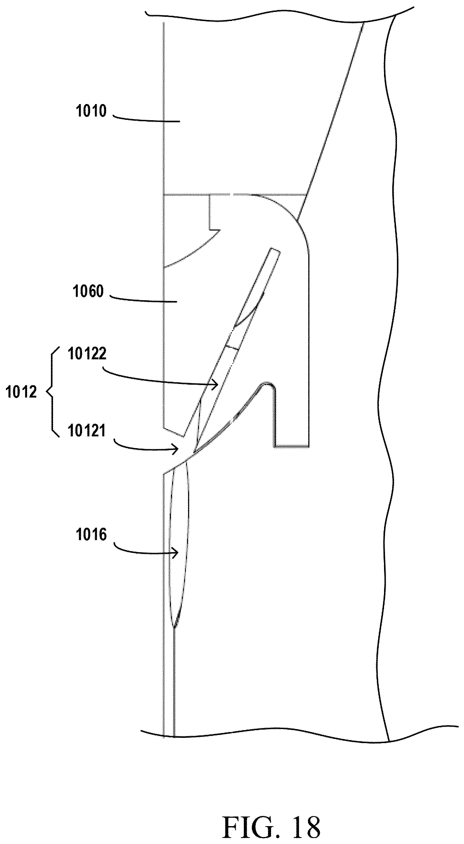

The suture slot includes a first slot portion and a second slot portion. The first slot portion extends at least partially towards the axis from the outside to the second slot portion, and the second slot portion extends at least partially axially proximally from the first slot portion. In the view shown in FIG. 18, the extending length of the second slot portion is several times of the extending length of the first slot portion.

Referring back to FIG. 17, the first slot portion constitutes an elongated opening formed on the sidewall in a direction perpendicular to the longitudinal axis, and two ends of the elongated opening respectively extend proximally and towards the axis to jointly define the second slot portion.

The suture carrying portion of the access needle is concealed in the second slot portion. When a suture is to be loaded, the suture needs to move proximally to the suture carrying portion along the second slot portion. The distal end of the access needle carrying the suture can exit the main body housing via the needle exit hole. In the final step where the suture needs to be released, the suture also exits the main body housing via the integrated suture slot.

It should be noted that, except for the arrangement of the suture slot, various arrangements in this embodiment may be identical to or similar to those in the embodiment shown in FIGs. 1 to 11, and the description of the embodiment in FIGs. 1 to 11 should also be regarded as description of this embodiment.

It should be further noted that, specific arrangements in the foregoing embodiments may be combined to obtain new embodiments, and these new embodiments may also fall within the scope of the present invention as defined by the claims.

Also disclosed is a method of using the tissue closure device in any one of the foregoing solutions. The method is not explicitly claimed but it may be performed using tissue closure devices according to the invention The method includes a suture loading step and a needle exiting step. The suture loading step includes: loading a suture into a suture carrying portion of an access needle via a suture loading slot of a main body housing. The needle exiting step includes: causing a needle control firmware to drive the access needle loaded with the suture to extend outwardly via a needle exit hole of the main body housing to an actuated position, and this step is implemented by, for example, a needle control component.

From the above discussion, it can be seen that in the present invention, the needle control component controls the access needle in two main aspects: control of the orientation of the access needle in an initial position, which enables at least partial communication between the suture carrying portion of the access needle and the suture slot to allow loading of the suture into the suture carrying portion via the suture slot; and control of the actuation depth and the orientation of the access needle in a moving process from the initial position to the actuated position, which enables the access needle to accurately reach the actuated position at a predetermined depth and radial position.

A tissue closure device and an access needle of the present invention form an integrated unit which can be delivered to an operator conveniently. In the present invention, the needle control component can achieve accurate control over the retention and actuation processes of the access needle as such an integrated unit, such that an operator can conveniently load a suture when the access needle is held in the initial position in a predetermined orientation; and the access needle can be accurately actuated to a predetermined depth in the process of being actuated, to accurately deliver the suture to a predetermined position. The entire operating process is fast and a better operation effect can be achieved.

From the above, those skilled in the art will readily recognize that alternative structures of the structures disclosed in the present invention may be used as feasible alternative embodiments, and the embodiments disclosed herein may be combined to form new embodiments, which may also fall within the scope of the appended claims.

## Claims

1. A tissue closure device (100), comprising:
a main body housing (10; 610; 710; 810; 1010) having a longitudinal axis, the main body housing (10; 610; 710; 810; 1010) internally defining a chamber extending along the longitudinal axis, and at least two suture slots (12; 2012) and at least two needle exit holes (16; 616; 716; 816; 916; 1016) being provided on a sidewall of the main body housing (10; 610; 710; 810; 1010); and
at least two access needles (50; 1050), the at least two access needles (50; 1050) capable of being preloaded in the chamber and provided with a suture carrying portion (51; 1051) close to a tip (52) thereof, the at least two access needles (50; 1050) being formed with a mating portion (55); and
a needle control component (20), the needle control component (20) being mounted to the main body housing (10; 610; 710; 810; 1010) and axially movable relative to the main body housing (10; 610; 710; 810; 1010), the needle control component (20) being directly or indirectly connected to the at least two access needles (50; 1050) by the mating portion (55) to hold the at least two access needles (50; 1050) in a predetermined orientation and to control the at least two access needles (50; 1050) such that the needle control component (20) actuates the at least two access needles (50; 1050) simultaneously, wherein:
the needle control component (20) is capable of holding the at least two access needles (50; 1050) in the initial position in the predetermined orientation, in the initial position the at least two access needles (50; 1050) are located within the chamber, and the suture carrying portion (51; 1051) of the at least two access needles (50; 1050) in the predetermined orientation at least partially communicates with the respective suture slot (12; 2012) so as to allow a suture to be loaded to the respective suture carrying portion via the respective suture slot (12; 2012); and
the needle control component (20) is capable of driving the at least two access needles (50; 1050) to be actuated from the initial position to an actuated position at a predetermined depth, in the actuated position, a tip (552) of each of the at least two access needles (50; 1050) extends out of the main body housing (10; 610; 710; 810; 1010) via the a respective one of the at least two needle exit holes (16; 616; 716; 816; 916; 1016).

2. The tissue closure device according to claim 1, wherein a position-limiting structure is provided at the connection position between the needle control component (20) and the main body housing (10; 610; 710; 810; 1010), and the position-limiting structure only allows the needle control component (20) to move axially relative to the main body housing (10; 610; 710; 810; 1010).

3. The tissue closure device according to claim 1 or 2, wherein the needle control component (20) has a needle operation portion (21) located outside the main body housing (10; 610; 710; 810; 1010) for a user to operate, and a needle actuation portion (22) connected to the at least two access needles (50; 1050) and at least partially located in the chamber.

4. The tissue closure device according to claim 3, wherein the position-limiting structure comprises an axially extending position-limiting slot (224) located on an outer surface of the needle actuation portion (22), and a protrusion located on an inner surface of the main body housing (10; 610; 710; 810; 1010) and having a width matching that of the position-limiting slot (224).

5. The tissue closure device according to claim 4, wherein the protrusion slides in the position-limiting slot (224) so that the needle control component (20) moves from a first position to a second position relative to the main body housing (10; 610; 710; 810; 1010), when the needle control component (20) is located at the first position, the needle control component holds (20) the at least two access needles (50; 1050) in the initial position; and in the process of the needle control component (20) moving from the first position to the second position, the needle control component (20) actuates the at least two access needles (50; 1050) to the actuated position.

6. The tissue closure device according to claim 4, wherein the main body housing (10; 610; 710; 810; 1010) comprises a left half housing (14) and a right half housing (15), the left half housing (14) and the right half housing (15) are butted together along a direction transverse to the longitudinal axis, wherein the left half housing (14) and the right half housing (15) are respectively provided with protruding halves at positions thereof adjacent to each other, and the protruding halves of the left half housing (14) and the right half housing (15) are butted together to jointly define the protrusion.

7. The tissue closure device according to claim 1, wherein a respective wall defining each suture slot (12; 1012) partially comprises a flat surface, the at least two access needles (50; 1050) being controlled such that when located in the initial position, the at least two access needles (50; 1050) are fit to the flat surface and prevented from rotating in the respective suture slot (12; 1012).

8. The tissue closure device according to claim 1, wherein the needle control component (20) has an axially extending mounting hole (222) for each of the at least two access needles, the inner diameter of each mounting hole (222) matches the outer diameter of the respective access needle (50; 1050), and the needle control component (20) is provided with a fixing pin (223) therein transverse to the longitudinal axis, the fixing pin (223) being engaged and fixed to the mating portion (55) of the at least two access needles (50; 1050), wherein optionally
the needle control component (20) is provided with two mounting holes (222) that are symmetrical about a longitudinal axis for correspondingly mounting two access needles (50; 1050), and the fixing pin (223) is connected between the two mounting holes (222) and joined to the two access needles (50; 1050), respectively.

9. The tissue closure device according to claim 1, wherein the needle control component (20) is directly molded together with the mating portion of each of the at least two access needles (50; 1050).

10. The tissue closure device according to any one of claims 1-9, wherein the mating portion (55) of each of the at least two access needles (50; 1050) is a notch (55), the notch (55) having a flat surface parallel to the axis of the respective access needle (50; 1050).

11. The tissue closure device according to claim 1, wherein the tissue closure device comprises a stabilizer (40) mounted at one end of the main body housing (10; 610; 710; 810; 1010) and actuatable between a folded position and an unfolded position, and the tissue closure device further comprises a stabilizer control component (30), the stabilizer control component (30) being partially located within the chamber and connected to the stabilizer (40), the stabilizer control component (30) being axially movable relative to the main body housing (10; 610; 710; 810; 1010) to actuate the stabilizer (40) from the folded position to the unfolded position.

12. The tissue closure device according to claim 11, wherein the sidewall of the main body housing (10; 610; 710; 810; 1010) is provided with a first window (101) and a second window (102), and the stabilizer control component (30) comprises:
a stabilizer operation portion (31) exposed through the first window (101), the stabilizer operation portion (31) being axially slidable in the first window (101), and
a positioning protrusion (32) fixed relative to the stabilizer operation portion (31), an outer profile of the positioning protrusion (32) matching the second window (102), such that when the positioning protrusion (32) is placed in the second window (102), the stabilizer control component (30) is locked relative to the main body housing (10; 610; 710; 810; 1010).

13. The tissue closure device according to claim 12, wherein the needle control component (20) is also partially positioned within the chamber and a recessed section (221) is provided on a sidewall of the needle control component (20), at least the stabilizer operation portion (31) being correspondingly mounted at the recessed section (221).

14. The tissue closure device according to claim 13, wherein the stabilizer control component (30) comprises two stabilizer operation portions (31) that are symmetrical about the longitudinal axis, and the stabilizer control component (30) further comprises a stabilizer actuation portion (33) fixedly connected to the two stabilizer operation portions (31), wherein the needle control component (20) is partially located between the two stabilizer operation portions (31) and completely located on the side of the stabilizer actuation portion (33) away from the at least two needle exit holes (16; 616; 716; 816; 916; 1016).

15. The tissue closure device according to any one of claims 1-9, wherein the at least two access needles (50; 1050) are made of a plastic material, and the chamber comprises a needle channel (17) for each of the at least two access needles (50; 1050) to move in a predetermined posture.

16. The tissue closure device according to claim 15, wherein each needle channel (17) comprises at least a curved segment and optionally a straight segment parallel to the longitudinal axis, wherein the curved segment optionally extends partially about the longitudinal axis.

17. The tissue closure device according to claim 15, wherein the number of the needle channels (17) is two, and the two needle channels (17) are provided such that: for any axial position, the segments of the two needle channels (17) at the axial position are symmetrical about the longitudinal axis.

18. The tissue closure device according to any one of claims 1-9, wherein each suture slot (12) comprises a suture loading slot (121; 6121; 7121; 8121; 9121) and a suture releasing slot (122; 6122; 7122; 8122; 9122), the suture loading slot (121; 6121; 7121; 8121; 9121) being configured to allow the suture to be loaded from the outside of the tissue closure device into the suture carrying portion (51; 1051) of the respective access needle (50; 1050) via the suture loading slot (121; 6121; 7121; 8121; 9121), and the suture releasing slot (122; 6122; 7122; 8122; 9122) being connected between the suture loading slot (121; 6121; 7121; 8121; 9121) and the respective needle exit hole (16; 616; 716; 816; 916; 1016) and configured to allow the suture to disengage from the tissue closure device via the suture releasing slot (122; 6122; 7122; 8122; 9122), wherein the suture loading slot (121; 6121; 7121; 8121; 9121) and the respective suture carrying portion (51; 1051) are at least partially aligned when the respective access needle (50; 1050) is in the initial position.

19. The tissue closure device according to any one of claims 1-9, wherein each suture slot (1012) is an integrated slot structure serving as both a suture loading slot and a suture releasing slot, each suture slot (1012) being configured to allow the suture to be loaded from the outside of the tissue closure device into the respective suture carrying portion (1051) of the respective access needle (1050) via the respective suture slot (1012) and to allow the suture to disengage from the tissue closure device via the respective suture slot (1012).

20. The tissue closure device according to claim 19, wherein the suture loading slot (1012) has a first slot portion (10121) and a second slot portion (10122), the first slot portion (10121) extending at least partially towards the axis from the outside to the second slot portion (10122), and the second slot portion (10122) extending at least partially axially from the first slot (10121) portion along a direction away from the respective needle exit hole (1016), wherein the second slot portion (10122) and the suture carrying portion (1051) are at least partially aligned.

21. The tissue closure device according to claim 20, wherein either:
the first slot portion (10121) constitutes an elongated opening along a direction at least partially perpendicular to the longitudinal axis, two terminal ends of the elongated opening respectively extending along a direction away from the respective needle exit hole (1016) and towards the axis to jointly define the second slot portion (10122); or
the extending directions of the suture releasing slot and the second slot portion (10122) are the same.

22. The tissue closure device according to any one of claims 1-9, wherein a notch surface (618) that constitutes a guide structure is provided at an inlet of the suture slot on the sidewall of the main body housing (610).

23. The tissue closure device according to any one of claims 1-9, wherein each needle exit hole (16; 616; 716; 816; 916; 1016) is an opening matching the shape of the respective access needle (50; 1050).

24. The tissue closure device according to any one of claims 1-9, wherein the tissue closure device comprises at least two stabilizers (40) mounted at one end of the main body housing (10; 610; 710; 810; 1010) and actuatable between a folded position and an unfolded position, and in the process of the at least two access needles (50; 1050) moving from the initial portion to the actuated position, each of the at least two access needles (50; 1050) passes through a respective one of the at least two stabilizers (40) in the unfolded position.

## Patentansprüche

1. Gewebeverschlussvorrichtung (100), die umfasst:
ein Grundkörpergehäuse (10; 610; 710; 810; 1010), das eine Längsachse aufweist, wobei das Grundkörpergehäuse (10; 610; 710; 810; 1010) intern eine sich entlang der Längsachse erstreckende Kammer und mindestens zwei Nahtschlitze (12; 2012) und mindestens zwei Nadelaustrittslöcher (16; 616; 716; 816; 916; 1016) definiert, die an einer Seitenwand des Grundkörpergehäuses (10; 610; 710; 810; 1010) bereitgestellt werden; und
mindestens zwei Zugangsnadeln (50; 1050), wobei die mindestens zwei Zugangsnadeln (50; 1050) imstande sind, in der Kammer vorgeladen zu werden, und mit einem nahtführenden Abschnitt (51; 1051) nahe einer Spitze (52) davon bereitsgestellt sind, wobei die mindestens zwei Zugangsnadeln (50; 1050) mit einem Gegenabschnitt (55) ausgebildet sind; und
eine Nadelsteuerungskomponente (20), wobei die Nadelsteuerungskomponente (20) an dem Grundkörpergehäuse (10; 610; 710; 810; 1010) montiert und relativ zu dem Grundkörpergehäuse (10; 610; 710; 810; 1010) axial beweglich ist, wobei die Nadelsteuerungskomponente (20) durch den Gegenabschnitt (55) direkt oder indirekt mit den mindestens zwei Zugangsnadeln (50; 1050) verbunden ist, um die mindestens zwei Zugangsnadeln (50; 1050) in einer vorbestimmten Ausrichtung zu halten und um die mindestens zwei Zugangsnadeln (50; 1050) derart zu steuern, dass die Nadelsteuerungskomponente (20) die mindestens zwei Zugangsnadeln (50; 1050) gleichzeitig auslöst, wobei:
die Nadelsteuerungskomponente (20) imstande ist, die mindestens zwei Zugangsnadeln (50; 1050) in der Ausgangsposition in der vorbestimmten Ausrichtung zu halten, sich die mindestens zwei Zugangsnadeln (50; 1050) innerhalb der Kammer in der Ausgangsposition befinden und der nahtführende Abschnitt (51; 1051) der mindestens zwei Zugangsnadeln (50, 1050) in der vorbestimmten Ausrichtung mindestens teilweise mit dem jeweiligen Nahtschlitz (12; 2012) in Verbindung steht, um zu ermöglichen, dass eine Naht über den jeweiligen Nahtschlitz (12; 2012) in den jeweiligen nahtführenden Abschnitt geladen wird; und
die Nadelsteuerungskomponente (20) imstande ist, die mindestens zwei Zugangsnadeln (50; 1050) anzutreiben, um von der Ausgangsposition in eine ausgelöste Position in einer vorbestimmten Tiefe ausgelöst zu werden, wobei in der ausgelösten Position eine Spitze (552) jeder der mindestens zwei Zugangsnadeln (50; 1050) über ein jeweiliges der mindestens zwei Nadelaustrittslöcher (16; 616; 716; 816; 916; 1016) aus dem Grundkörpergehäuse (10; 610; 710; 810; 1010) herausragt.

2. Gewebeverschlussvorrichtung nach Anspruch 1, wobei eine positionsbegrenzende Struktur an der Verbindungsposition zwischen der Nadelsteuerungskomponente (20) und dem Grundkörpergehäuse (10; 610; 710; 810; 1010) bereitgestellt ist und die positionsbegrenzende Struktur der Nadelsteuerungskomponente (20) nur erlaubt, sich relativ zu dem Grundkörpergehäuse (10; 610; 710; 810; 1010) axial zu bewegen.

3. Gewebeverschlussvorrichtung nach Anspruch 1 oder 2, wobei die Nadelsteuerungskomponente (20) einen Nadelbetätigungsabschnitt (21), der sich außerhalb des Grundkörpergehäuses (10; 610; 710; 810; 1010) befindet, damit ein Benutzer ihn betätigen kann, und einen Nadelauslösungsabschnitt (22) aufweist, der mit den mindestens zwei Zugangsnadeln (50; 1050) verbunden ist und sich mindestens teilweise in der Kammer befindet.

4. Gewebeverschlussvorrichtung nach Anspruch 3, wobei die positionsbegrenzende Struktur einen sich axial erstreckenden positionsbegrenzenden Schlitz (224), der sich auf einer Außenoberfläche des Nadelauslösgungsabschnitts (22) befindet, und einen Vorsprung umfasst, der sich auf einer Innenoberfläche des Grundkörpergehäuses (10; 610; 710; 810; 1010) befindet und eine Breite aufweist, die der des positionsbegrenzenden Schlitzes (224) entspricht.

5. Gewebeverschlussvorrichtung nach Anspruch 4, wobei der Vorsprung in den positionsbegrenzenden Schlitz (224) gleitet, so dass sich die Nadelsteuerungskomponente (20) von einer ersten Position zu einer zweiten Position relativ zu dem Grundkörpergehäuse (10; 610; 710; 810; 1010) bewegt, wenn die Nadelsteuerungskomponente (20) sich in der ersten Position befindet, die Nadelsteuerungskomponente (20) die mindestens zwei Zugangsnadeln (50; 1050) in der Ausgangsposition hält; und bei dem Prozess eines Bewegens der Nadelsteuerungskomponente (20) von der ersten Position zu der zweiten Position die Nadelsteuerungskomponente (20) die mindestens zwei Zugangsnadeln (50; 1050) in die ausgelöste Position auslöst.

6. Gewebeverschlussvorrichtung nach Anspruch 4, wobei das Grundkörpergehäuse (10; 610; 710; 810; 1010) ein linkes Halbgehäuse (14) und ein rechtes Halbgehäuse (15) umfasst, das linke Halbgehäuse (14) und das rechte Halbgehäuse (15) entlang einer Richtung quer zu der Längsachse aneinanderstoßen, wobei das linke Halbgehäuse (14) und das rechte Halbgehäuse (15) jeweils mit vorstehenden Hälften an Positionen davon benachbart zueinander bereitgestellt wird, und die vorstehenden Hälften des linken Halbgehäuses (14) und des rechten Halbgehäuses (15) aneinanderstoßen, um gemeinsam den Vorsprung zu definieren.

7. Gewebeverschlussvorrichtung nach Anspruch 1, wobei eine entsprechende Wand, die jeden Nahtschlitz (12; 1012) definiert, teilweise eine flache Oberfläche umfasst, wobei die mindestens zwei Zugangsnadeln (50; 1050) derart gesteuert werden, dass, wenn sie sich in der Anfangsposition befinden, die mindestens zwei Zugangsnadeln (50; 1050) an die flache Oberfläche angepasst sind und daran gehindert werden, sich in dem jeweiligen Nahtschlitz (12; 1012) zu drehen.

8. Gewebeverschlussvorrichtung nach Anspruch 1, wobei die Nadelsteuerungskomponente (20) ein sich axial erstreckendes Montageloch (222) für jede der mindestens zwei Zugangsnadeln aufweist, der Innendurchmesser jedes Montagelochs (222) mit dem Außendurchmesser der jeweiligen Zugangsnadel (50; 1050) übereinstimmt und die Nadelsteuerungskomponente (20) mit einem Fixierstift (223) darin quer zu der Längsachse bereitgestellt wird, wobei der Fixierstift (223) mit dem Gegenabschnitt (55) der mindestens zwei Zugangsnadeln (50; 1050) in Eingriff steht und daran fixiert ist, wobei optional
die Nadelsteuerungskomponente (20) mit zwei Montagelöchern (222) bereitgestellt wird, die symmetrisch um eine Längsachse zum entsprechenden Montieren der zwei Zugangsnadeln (50; 1050) angeordnet sind, und der Fixierstift (223) zwischen den zwei Montagelöchern (222) verbunden und jeweils mit den zwei Zugangsnadeln (50; 1050) verknüpft ist.

9. Gewebeverschlussvorrichtung nach Anspruch 1, wobei die Nadelsteuerungskomponente (20) direkt mit dem Gegenabschnitt jeder der mindestens zwei Zugangsnadeln (50; 1050) zusammengeformt ist.

10. Gewebeverschlussvorrichtung nach einem der Ansprüche 1 bis 9, wobei der Gegenabschnitt (55) jeder der mindestens zwei Zugangsnadeln (50; 1050) eine Kerbe (55) ist, wobei die Kerbe (55) eine flache Oberfläche parallel zu der Achse der jeweiligen Zugangsnadel (50; 1050) aufweist.

11. Gewebeverschlussvorrichtung nach Anspruch 1, wobei die Gewebeverschlussvorrichtung einen Stabilisator (40) umfasst, der an einem Ende des Grundkörpergehäuses (10; 610; 710; 810; 1010) montiert und zwischen einer gefalteten Position und einer entfalteten Position auslösbar ist, und die Gewebeverschlussvorrichtung ferner eine Stabilisatorsteuerungskomponente (30) umfasst, wobei sich die Stabilisatorsteuerungskomponente (30) teilweise innerhalb der Kammer befindet und mit dem Stabilisator (40) verbunden ist, wobei die Stabilisatorsteuerungskomponente (30) relativ zu dem Grundkörpergehäuse (10; 610; 710; 810; 1010) axial beweglich ist, um den Stabilisator (40) von der gefalteten Position zu der entfalteten Position auszulösen.

12. Gewebeverschlussvorrichtung nach Anspruch 11, wobei die Seitenwand des Grundkörpergehäuses (10; 610; 710; 810; 1010) mit einem ersten Fenster (101) und einem zweiten Fenster (102) bereitgestellt wird, und die Stabilisatorsteuerungskomponente (30) umfasst:
einen Stabilisatorbetätigungsabschnitt (31), der durch das erste Fenster (101) freigelegt ist, der Stabilisatorbetätigungsabschnitt (31) in dem ersten Fenster (101) axial verschoben werden kann, und
einen Positionierungsvorsprung (32), der relativ zu dem Stabilisatorbetätigungsabschnitt (31) fixiert ist, wobei ein Außenprofil des Positionierungsvorsprungs (32) dem zweiten Fenster (102) derart entspricht, dass, wenn der Positionierungsvorsprung (32) in dem zweiten Fenster (102) platziert wird, die Stabilisatorsteuerungskomponente (30) relativ zu dem Grundkörpergehäuse (10; 610; 710; 810; 1010) verriegelt wird.

13. Gewebeverschlussvorrichtung nach Anspruch 12, wobei die Nadelsteuerungskomponente (20) auch teilweise innerhalb der Kammer positioniert ist und ein vertiefter Bereich (221) an einer Seitenwand der Nadelsteuerungskomponente (20) bereitgestellt ist, wobei mindestens der Stabilisatorbetätigungsabschnitt (31) entsprechend an dem vertieften Bereich (221) montiert ist.

14. Gewebeverschlussvorrichtung nach Anspruch 13, wobei die Stabilisatorsteuerungskomponente (30) zwei Stabilisatorbetätigungsabschnitte (31) umfasst, die symmetrisch bezüglich der Längsachse liegen und die Stabilisatorsteuerungskomponente (30) ferner einen Stabilisatorauslösungsabschnitt (33) umfasst, der fest mit den zwei Stabilisatorbetätigungsabschnitten (31) verbunden ist, wobei sich die Nadelsteuerungskomponente (20) teilweise zwischen den zwei Stabilisatorbetätigungsabschnitten (31) befindet und vollständig auf der Seite des Stabilisatorauslösungsabschnitts (33) angeordnet ist, die von den mindestens zwei Nadelaustrittslöchern (16; 616; 716; 816; 916; 1016) abgewandt ist.

15. Gewebeverschlussvorrichtung nach einem der Ansprüche 1 bis 9, wobei die mindestens zwei Zugangsnadeln (50; 1050) aus einem Kunststoffmaterial hergestellt sind, und die Kammer einen Nadelkanal (17) für jede der mindestens zwei Zugangsnadeln (50; 1050) umfasst, um sich in einer vorbestimmten Haltung zu bewegen.

16. Gewebeverschlussvorrichtung nach Anspruch 15, wobei jeder Nadelkanal (17) mindestens ein gekrümmtes Segment und optional ein gerades Segment parallel zu der Längsachse umfasst, wobei sich das gekrümmte Segment optional teilweise um die Längsachse erstreckt.

17. Gewebeverschlussvorrichtung nach Anspruch 15, wobei die Anzahl der Nadelkanäle (17) zwei beträgt, und die zwei Nadelkanäle (17) derart bereitgestellt sind, dass: für eine beliebige axiale Position, die Segmente der zwei Nadelkanäle (17) an der axialen Position bezüglich der Längsachse symmetrisch sind.

18. Gewebeverschlussvorrichtung nach einem der Ansprüche 1 bis 9, wobei jeder Nahtschlitz (12) einen Nahtbeladungsschlitz (121; 6121; 7121; 8121; 9121) und einen Nahtfreigabeschlitz (122; 6122; 7122; 8122; 9122) umfasst, wobei der Nahtbeladungsschlitz (121; 6121; 7121; 8121; 9121) konfiguriert ist, um zu ermöglichen, dass die Naht von der Außenseite der Gewebeverschlussvorrichtung in den nahtführenden Abschnitt (51; 1051) der jeweiligen Zugangsnadel (50; 1050) über den Nahtbeladungsschlitz (121; 6121; 7121; 8121; 9121) geladen wird, und der Nahtfreigabeschlitz (122; 6122; 7122; 8122; 9122) zwischen dem Nahtbeladungsschlitz (121; 6121; 7121; 8121; 9121) und dem jeweiligen Nadelaustrittsloch (16; 616; 716; 816; 916; 1016) verbunden ist und konfiguriert ist, um zu ermöglichen, dass sich die Naht von der Gewebeverschlussvorrichtung über den Nahtfreigabeschlitz (122; 6122; 7122; 8122; 9122) löst, wobei der Nahtbeladungsschlitz (121; 6121; 7121; 8121; 9121) und der jeweilige nahtführende Abschnitt (51; 1051) mindestens teilweise ausgerichtet sind, wenn sich die jeweilige Zugangsnadel (50; 1050) in der Anfangsposition befindet.

19. Gewebeverschlussvorrichtung nach einem der Ansprüche 1 bis 9, wobei jeder Nahtschlitz (1012) eine integrierte Schlitzstruktur ist, die sowohl als ein Nahtbeladungsschlitz als auch ein Nahtfreigabeschlitz dient, wobei jeder Nahtschlitz (1012) konfiguriert ist, um zu ermöglichen, dass die Naht von außerhalb der Gewebeverschlussvorrichtung in den jeweiligen nahtführenden Abschnitt (1051) der jeweiligen Zugangsnadel (1050) über den jeweiligen Nahtschlitz (1012) geladen wird, und um zu ermöglichen, dass sich die Naht von der Gewebeverschlussvorrichtung über den jeweiligen Nahtschlitz (1012) löst.

20. Gewebeverschlussvorrichtung nach Anspruch 19, wobei der Nahtbeladungsschlitz (1012) einen ersten Schlitzabschnitt (10121) und einen zweiten Schlitzabschnitt (10122) aufweist, wobei sich der erste Schlitzabschnitt (10121) mindestens teilweise zu der Achse von außen zu dem zweiten Schlitzabschnitt (10122) hin erstreckt und sich der zweite Schlitzabschnitt (10122) mindestens teilweise axial von dem ersten Schlitz(10121)-Abschnitt entlang einer Richtung weg von dem jeweiligen Nadelaustrittsloch (1016) erstreckt, wobei der zweite Schlitzabschnitt (10122) und der nahtführende Abschnitt (1051) mindestens teilweise ausgerichtet sind.

21. Gewebeverschlussvorrichtung nach Anspruch 20, wobei entweder:
der erste Schlitzabschnitt (10121) eine längliche Öffnung entlang einer Richtung bildet, die mindestens teilweise senkrecht zu der Längsachse verläuft, wobei sich zwei Anschlussenden der länglichen Öffnung jeweils entlang einer Richtung weg von dem jeweiligen Nadelaustrittsloch (1016) und zu der Achse hin erstrecken, um gemeinsam den zweiten Schlitzabschnitt (10122) zu definieren; oder
die Erstreckungsrichtungen des Nahtfreigabeschlitzes und des zweiten Schlitzabschnitts (10122) gleich sind.

22. Gewebeverschlussvorrichtung nach einem der Ansprüche 1 bis 9, wobei eine Kerboberfläche (618), die eine Führungsstruktur bildet, an einem Einlass des Nahtschlitzes an der Seitenwand des Grundkörpergehäuses (610) bereitgestellt ist.

23. Gewebeverschlussvorrichtung nach einem der Ansprüche 1 bis 9, wobei jedes Nadelaustrittsloch (16; 616; 716; 816; 916; 1016) eine Öffnung ist, die der Form der jeweiligen Zugangsnadel (50; 1050) entspricht.

24. Gewebeverschlussvorrichtung nach einem der Ansprüche 1 bis 9, wobei die Gewebeverschlussvorrichtung mindestens zwei Stabilisatoren (40) umfasst, die an einem Ende des Grundkörpergehäuses (10; 610; 710; 810; 1010) montiert und zwischen einer gefalteten Position und einer entfalteten Position ausgelöst werden können, und bei dem Prozess des Bewegebs der mindestens zwei Zugangsnadeln (50; 1050) von dem anfänglichen Abschnitt zu der ausgelösten Position, jede der mindestens zwei Zugangsnadeln (50; 1050) durch einen jeweiligen der mindestens zwei Stabilisatoren (40) in der entfalteten Position hindurchgeht.

## Revendications

1. Dispositif de fermeture de tissu (100), comprenant :
un logement de corps principal (10 ; 610 ; 710 ; 810 ; 1010) ayant un axe longitudinal, le logement de corps principal (10 ; 610 ; 710 ; 810 ; 1010) définissant de façon interne une chambre s'étendant le long de l'axe longitudinal, et au moins deux fentes de suture (12 ; 2012) et au moins deux trous de sortie d'aiguille (16 ; 616 ; 716 ; 816 ; 916 ; 1016) étant fournis sur une paroi latérale du logement de corps principal (10 ; 610 ; 710 ; 810 ; 1010) ; et
au moins deux aiguilles d'accès (50 ; 1050), les au moins deux aiguilles d'accès (50 ; 1050) étant capables d'être préchargées dans la chambre et pourvues d'une partie de support de suture (51 ; 1051) près d'une pointe (52) de celles-ci, les au moins deux aiguilles d'accès (50 ; 1050) étant formées avec une partie d'accouplement (55) ; et
un composant de commande d'aiguille (20), le composant de commande d'aiguille (20) étant monté sur le logement de corps principal (10 ; 610 ; 710 ; 810 ; 1010) et déplaçable axialement par rapport au logement de corps principal (10 ; 610 ; 710 ; 810 ; 1010), le composant de commande d'aiguille (20) étant relié directement ou indirectement aux au moins deux aiguilles d'accès (50 ; 1050) par la partie d'accouplement (55) pour maintenir les au moins deux aiguilles d'accès (50 ; 1050) dans une orientation prédéterminée et pour commander les au moins deux aiguilles d'accès (50 ; 1050) de telle sorte que le composant de commande d'aiguille (20) actionne les au moins deux aiguilles d'accès (50 ; 1050) simultanément, dans lequel :
le composant de commande d'aiguille (20) est capable de maintenir les au moins deux aiguilles d'accès (50 ; 1050) dans la position initiale dans l'orientation prédéterminée, dans la position initiale les au moins deux aiguilles d'accès (50 ; 1050) sont localisées au sein de la chambre, et la partie de support de suture (51 ; 1051) des au moins deux aiguilles d'accès (50, 1050) dans l'orientation prédéterminée communique au moins partiellement avec la fente de suture (12 ; 2012) respective de façon à permettre à une suture d'être chargée sur la partie de support de suture respective par l'intermédiaire de la fente de suture (12 ; 2012) respective ; et
le composant de commande d'aiguille (20) est capable d'entraîner les au moins deux aiguilles d'accès (50 ; 1050) pour qu'elles soient actionnées de la position initiale à une position actionnée à une profondeur prédéterminée, dans la position actionnée, une pointe (552) de chacune des au moins deux aiguilles d'accès (50 ; 1050) s'étend à l'extérieur du logement de corps principal (10 ; 610 ; 710 ; 810 ; 1010) par l'intermédiaire d'un respectif des au moins deux trous de sortie d'aiguille (16 ; 616 ; 716 ; 816 ; 916 ; 1016).

2. Dispositif de fermeture de tissu selon la revendication 1, dans lequel une structure de limitation de position est fournie au niveau d'une position de liaison entre le composant de commande d'aiguille (20) et le logement de corps principal (10 ; 610 ; 710 ; 810 ; 1010), et la structure de limitation de position permet uniquement au composant de commande d'aiguille (20) de se déplacer axialement par rapport au logement de corps principal (10 ; 610 ; 710 ; 810 ; 1010).

3. Dispositif de fermeture de tissu selon la revendication 1 ou 2, dans lequel le composant de commande d'aiguille (20) a une partie de fonctionnement d'aiguille (21) localisée à l'extérieur du logement de corps principal (10 ; 610 ; 710 ; 810 ; 1010) permettant un fonctionnement par un utilisateur, et une partie d'actionnement d'aiguille (22) reliée aux au moins deux aiguilles d'accès (50 ; 1050) et au moins partiellement localisée dans la chambre.

4. Dispositif de fermeture de tissu selon la revendication 3, dans lequel la structure de limitation de position comprend une fente de limitation de position (224) s'étendant axialement localisée sur une surface externe de la partie d'actionnement d'aiguille (22), et une saillie localisée sur une surface interne du logement de corps principal (10 ; 610 ; 710 ; 810 ; 1010) et ayant une largeur concordant avec celle de la fente de limitation de position (224).

5. Dispositif de fermeture de tissu selon la revendication 4, dans lequel la saillie coulisse dans la fente de limitation de position (224) de sorte que le composant de commande d'aiguille (20) se déplace d'une première position à une seconde position par rapport au logement de corps principal (10 ; 610 ; 710 ; 810 ; 1010), lorsque le composant de commande d'aiguille (20) est localisé au niveau de la première position, le composant de commande d'aiguille maintient (20) les au moins deux aiguilles d'accès (50 ; 1050) dans la position initiale ; et dans le processus du composant de commande d'aiguille (20) se déplaçant de la première position à la seconde position, le composant de commande d'aiguille (20) actionne les au moins deux aiguilles d'accès (50 ; 1050) à la position actionnée.

6. Dispositif de fermeture de tissu selon la revendication 4, dans lequel le logement de corps principal (10 ; 610 ; 710 ; 810 ; 1010) comprend un demi-logement gauche (14) et un demi-logement droit (15), le demi-logement gauche (14) et le demi-logement droit (15) sont mis bout à bout le long d'une direction transversale à l'axe longitudinal, dans lequel le demi-logement gauche (14) et le demi-logement droit (15) sont respectivement pourvus de moitiés en saillie au niveau de positions de celles-ci adjacentes l'une à l'autre, et les moitiés en saillie du demi-logement gauche (14) et du demi-logement droit (15) sont mises bout à bout pour définir conjointement la saillie.

7. Dispositif de fermeture de tissu selon la revendication 1, dans lequel une paroi respective définissant chaque fente de suture (12 ; 1012) comprend partiellement une surface plane, les au moins deux aiguilles d'accès (50 ; 1050) étant commandées de telle sorte que lorsqu'elles sont localisées dans la position initiale, les au moins deux aiguilles d'accès (50 ; 1050) sont ajustées à la surface plane et empêchées de se mettre en rotation dans la fente de suture (12 ; 1012) respective.

8. Dispositif de fermeture de tissu selon la revendication 1, dans lequel le composant de commande d'aiguille (20) a un trou de montage (222) s'étendant axialement pour chacune des au moins deux aiguilles d'accès, le diamètre interne de chaque trou de montage (222) concorde avec le diamètre externe de l'aiguille d'accès (50 ; 1050) respective, et le composant de commande d'aiguille (20) est pourvu d'une broche de fixation (223) dans celui-ci transversal à l'axe longitudinal, la broche de fixation (223) étant en prise et fixée à la partie d'accouplement (55) des au moins deux aiguilles d'accès (50 ; 1050), dans lequel facultativement
le composant de commande d'aiguille (20) est pourvu de deux trous de montage (222) qui sont symétriques autour d'un axe longitudinal pour un montage en correspondance des deux aiguilles d'accès (50 ; 1050), et la broche de fixation (223) est reliée entre les deux trous de montage (222) et jointe aux deux aiguilles d'accès (50 ; 1050), respectivement.

9. Dispositif de fermeture de tissu selon la revendication 1, dans lequel le composant de commande d'aiguille (20) est directement moulé conjointement avec la partie d'accouplement de chacune des au moins deux aiguilles d'accès (50 ; 1050).

10. Dispositif de fermeture de tissu selon l'une quelconque des revendications 1 à 9, dans lequel la partie d'accouplement (55) de chacune des au moins deux aiguilles d'accès (50 ; 1050) est une encoche (55), l'encoche (55) ayant une surface plane parallèle à l'axe de l'aiguille d'accès (50 ; 1050) respective.

11. Dispositif de fermeture de tissu selon la revendication 1, dans lequel le dispositif de fermeture de tissu comprend un stabilisateur (40) monté au niveau d'une extrémité du logement de corps principal (10 ; 610 ; 710 ; 810 ; 1010) et actionnable entre une position pliée et une position dépliée, et le dispositif de fermeture de tissu comprend en outre un composant de commande de stabilisateur (30), le composant de commande de stabilisateur (30) étant partiellement localisé au sein de la chambre et relié au stabilisateur (40), le composant de commande de stabilisateur (30) étant déplaçable axialement par rapport au logement de corps principal (10 ; 610 ; 710 ; 810 ; 1010) pour actionner le stabilisateur (40) de la position pliée à la position dépliée.

12. Dispositif de fermeture de tissu selon la revendication 11, dans lequel la paroi latérale du logement de corps principal (10 ; 610 ; 710 ; 810 ; 1010) est pourvue d'une première fenêtre (101) et d'une seconde fenêtre (102), et le composant de commande de stabilisateur (30) comprend :
une partie de fonctionnement de stabilisateur (31) exposée à travers la première fenêtre (101), la partie de fonctionnement de stabilisateur (31) pouvant coulisser axialement dans la première fenêtre (101), et
une saillie de positionnement (32) fixe par rapport à la partie de fonctionnement de stabilisateur (31), un profil externe de la saillie de positionnement (32) concordant avec la seconde fenêtre (102), de telle sorte que lorsque la saillie de positionnement (32) est placée dans la seconde fenêtre (102), le composant de commande de stabilisateur (30) est verrouillé par rapport au logement de corps principal (10 ; 610 ; 710 ; 810 ; 1010).

13. Dispositif de fermeture de tissu selon la revendication 12, dans lequel le composant de commande d'aiguille (20) est également positionné partiellement au sein de la chambre et une section renfoncée (221) est fournie sur une paroi latérale du composant de commande d'aiguille (20), au moins la partie de fonctionnement de stabilisateur (31) étant montée de manière correspondante au niveau de la section renfoncée (221).

14. Dispositif de fermeture de tissu selon la revendication 13, dans lequel le composant de commande de stabilisateur (30) comprend deux parties de fonctionnement de stabilisateur (31) qui sont symétriques autour de l'axe longitudinal, et le composant de commande de stabilisateur (30) comprend en outre une partie d'actionnement de stabilisateur (33) reliée fixement aux deux parties de fonctionnement de stabilisateur (31), dans lequel le composant de commande d'aiguille (20) est partiellement localisé entre les deux parties de fonctionnement de stabilisateur (31) et complètement localisé sur le côté de la partie d'actionnement de stabilisateur (33) à l'écart des au moins deux trous de sortie d'aiguille (16 ; 616 ; 716 ; 816 ; 916 ; 1016).

15. Dispositif de fermeture de tissu selon l'une quelconque des revendications 1 à 9, dans lequel les au moins deux aiguilles d'accès (50 ; 1050) sont fabriquées dans un matériau en plastique, et la chambre comprend un canal d'aiguille (17) permettant à chacune des au moins deux aiguilles d'accès (50 ; 1050) de se déplacer dans une posture prédéterminée.

16. Dispositif de fermeture de tissu selon la revendication 15, dans lequel chaque canal d'aiguille (17) comprend au moins un segment courbé et facultativement un segment rectiligne parallèle à l'axe longitudinal, dans lequel le segment courbé s'étend facultativement partiellement autour de l'axe longitudinal.

17. Dispositif de fermeture de tissu selon la revendication 15, dans lequel le nombre des canaux d'aiguille (17) est de deux, et les deux canaux d'aiguille (17) sont fournis de telle sorte que : pour l'une quelconque position axiale, les segments des deux canaux d'aiguille (17) au niveau de la position axiale sont symétriques autour de l'axe longitudinal.

18. Dispositif de fermeture de tissu selon l'une quelconque des revendications 1 à 9, dans lequel chaque fente de suture (12) comprend une fente de chargement de suture (121 ; 6121 ; 7121 ; 8121 ; 9121) et une fente de libération de suture (122 ; 6122 ; 7122 ; 8122 ; 9122), la fente de chargement de suture (121 ; 6121 ; 7121 ; 8121 ; 9121) étant conçue pour permettre à la suture d'être chargée depuis l'extérieur du dispositif de fermeture de tissu dans la partie de support de suture (51 ; 1051) de l'aiguille d'accès (50 ; 1050) respective par l'intermédiaire de la fente de chargement de suture (121 ; 6121 ; 7121 ; 8121 ; 9121), et la fente de libération de suture (122 ; 6122 ; 7122 ; 8122 ; 9122) étant reliée entre la fente de chargement de suture (121 ; 6121 ; 7121 ; 8121 ; 9121) et le trou de sortie d'aiguille (16 ; 616 ; 716 ; 816 ; 916 ; 1016) respectif et conçue pour permettre à la suture de se désaccoupler du dispositif de fermeture de tissu par l'intermédiaire de la fente de libération de suture (122 ; 6122 ; 7122 ; 8122 ; 9122), dans lequel la fente de chargement de suture (121 ; 6121 ; 7121 ; 8121 ; 9121) et la partie de support de suture (51 ; 1051) respective sont au moins partiellement alignées lorsque l'aiguille d'accès (50 ; 1050) respective est dans la position initiale.

19. Dispositif de fermeture de tissu selon l'une quelconque des revendications 1 à 9, dans lequel chaque fente de suture (1012) est une structure de fente intégrée servant à la fois de fente de chargement de suture et de fente de libération de suture, chaque fente de suture (1012) étant conçue pour permettre à la suture d'être chargée depuis l'extérieur du dispositif de fermeture de tissu dans la partie de support de suture (1051) respective de l'aiguille d'accès (1050) respective par l'intermédiaire de la fente de suture (1012) respective et pour permettre à la suture de se désaccoupler du dispositif de fermeture de tissu par l'intermédiaire de la fente de suture (1012) respective.

20. Dispositif de fermeture de tissu selon la revendication 19, dans lequel la fente de chargement de suture (1012) a une première partie de fente (10121) et une seconde partie de fente (10122), la première partie de fente (10121) s'étendant au moins partiellement en direction de l'axe depuis l'extérieur vers la seconde partie de fente (10122), et la seconde partie de fente (10122) s'étendant au moins partiellement axialement à partir de la première partie de fente (10121) le long d'une direction à l'écart du trou de sortie d'aiguille (1016) respectif, dans lequel la seconde partie de fente (10122) et la partie de support de suture (1051) sont au moins partiellement alignées.

21. Dispositif de fermeture de tissu selon la revendication 20, dans lequel soit :
la première partie de fente (10121) constitue une ouverture allongée le long d'une direction au moins partiellement perpendiculaire à l'axe longitudinal, deux extrémités terminales de l'ouverture allongée s'étendant respectivement le long d'une direction à l'écart du trou de sortie d'aiguille (1016) respectif et en direction de l'axe pour définir conjointement la seconde partie de fente (10122) ; soit
les directions d'extension de la fente de libération de suture et de la seconde partie de fente (10122) sont identiques.

22. Dispositif de fermeture de tissu selon l'une quelconque des revendications 1 à 9, dans lequel une surface d'encoche (618) qui constitue une structure de guidage est fournie au niveau d'une entrée de la fente de suture sur la paroi latérale du logement de corps principal (610).

23. Dispositif de fermeture de tissu selon l'une quelconque des revendications 1 à 9, dans lequel chaque trou de sortie d'aiguille (16 ; 616 ; 716 ; 816 ; 916 ; 1016) est une ouverture concordant avec la forme de l'aiguille d'accès (50 ; 1050) respective.

24. Dispositif de fermeture de tissu selon l'une quelconque des revendications 1 à 9, dans lequel le dispositif de fermeture de tissu comprend au moins deux stabilisateurs (40) montés au niveau d'une extrémité du logement de corps principal (10 ; 610 ; 710 ; 810 ; 1010) et actionnables entre une position pliée et une position dépliée, et dans le processus des au moins deux aiguilles d'accès (50 ; 1050) se déplaçant de la partie initiale à la position actionnée, chacune des au moins deux aiguilles d'accès (50 ; 1050) passe à travers un respectif des au moins deux stabilisateurs (40) dans la position dépliée.
